# EUROPEAN PATENT APPLICATION

(11) **EP 2 289 962 A2**
(43) Date of publication of application: **02.03.2011**
(21) Application number: 10182915.8
(22) Date of filing: 16.05.2005
(51) Int. Cl.: C08G 18/50, C08G 18/32, C08K 5/353, C08L 75/02, C07C 251/24, C07C 205/37, C07C 217/84, C07C 229/60, C07C 205/59, C07C 233/78

(54) **Polyurea compositions and compounds for the preparation thereof**

(30) Priority: 14.05.2004 US 570897 P
(62) Divisional of application: 05750361.7
(71) Applicant: Sawyer, Kenneth I., Sun City West AZ 85375 (US)
(72) Inventor: Sawyer, Kenneth, I., Sun City West, AZ 85375 (US); Kupper, Robert, J., East Greenwich, RI 02818 (US)
(74) Representative: Hamer, Christopher K.

(57) **Abstract**

The invention is directed to novel compounds that are used in the formation of polyurea compositions, including one-part and two-part polyurea compositions. The invention is also directed to polyureas formed from such compounds, cross-linked polyureas, and UV-stabilized polyureas.

## Description

### BACKGROUND OF THE INVENTION

### Field of the Invention

The invention relates to compounds that are used in the formation of polyurea compositions, to polyurea compositions using such compounds, and methods of their preparation and use.

### Related Art

U.S. Pat. No. 4,328,322 to Baron illustrates "two-part" or two component polyureas prepared by reaction of polyisocyanate and oligomeric aminobenzoic acid esters or amides.

U.S. Pat. No. 6,552,155 to Gutman et al. discloses polyaldimines of oligomeric aminobenzoic acid derivatives, and their use for preparation of moisture-curable, storage-stable, "one-part" polyureas. The polyureas disclosed in Gutman *et al.* are useful for a variety of coatings, including for example adhesives, paints, and sealing and waterproofing materials.

Despite the usefulness of known one-part and two-part compositions, it would be advantageous to employ polyureas with increased toughness and durability without a significant loss of flexibility of the material, improved stability, including UV stability, and/or utility for microbial or other biofouling in underwater applications.

### BRIEF SUMMARY OF THE INVENTION

The invention provides novel compounds (including those having Formulas IV, XI, and XII, described below) that can be used as a component in two-part polyureas, or as an intermediate for making novel imines. The imines can be subsequently used, *inter alia,* for preparing novel one-part polyureas. The invention also provides a novel intermediate compound having Formula V, described below for making a compound having Formula IV.

The invention also provides novel imine compounds (including those having Formulas VI, VII, XIII, and XIV, described below) that can be used, *inter alia,* for preparing novel one-part polyureas.

The invention additionally provides novel polyurea compositions, including one-part and two-part polyurea compositions, that incorporate the novel compounds described above.

The invention further provides novel compounds that can be used, *inter alia,* as cross-linking agents, including pentaerythritol-(4,3)-ethoxylatetetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, and 1,7-di(4-aminophenylcarbonyl)-1,4,7-triazaheptane. The invention also provides a novel intermediate, pentaerythritol-(4,3)-ethoxylate-tetrakis(4-nitrobenzoate), for preparing pentaerythritol-(4,3)-ethoxylate-tetrakis(4-aminobenzoate).

The invention also provides novel cross-linked polyureas, wherein a polyurea is cross-linked with one or more of pentaerythritol-(4,3)-ethoxylatetetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, 1,7-di(4-aminophenylcarbonyl)-1,4,7-triazaheptane; pentaerythritol tetrakis(2-aminobenzoate), pentaerythritol tetrakis(3-aminobenzoate), pentaerythritol tetrakis(4-aminobenzoate), or derivatives thereof. The invention further provides novel polyurea prepolymer compositions containing one or more of these cross-linking agents, or derivatives thereof.

The invention additionally provides novel UV-stabilized polyurea compositions comprising a polyurea and diphenyloxazole in an amount effective to provide protection against UV degradation. The invention further provides novel polyurea prepolymer compositions containing diphenyloxazole in an amount effective to provide protection against UV degradation when the polyurea prepolymer compositions are cured.

### DETAILED DESCRIPTION OF THE INVENTION

The invention provides novel polyurea compositions and compounds that can be used in the preparation such compositions, and methods of preparation of such compounds and compositions.

### Compounds of the Invention

The invention provides novel compounds can be used for the preparation of polyurea compositions, or as intermediates.

One aspect of the invention is directed to a compound having Formula I: 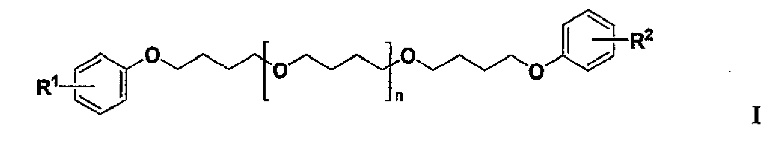 wherein n is from 0 to 30;
R¹ and R² are independently NH₂ or NO₂, or R¹ and R² are represented by Formulas II and III, respectively: 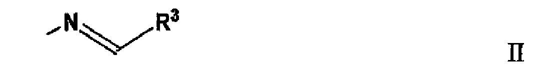 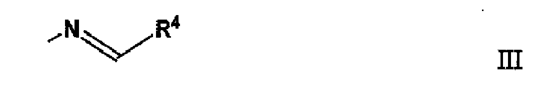 wherein R³ and R⁴ are independently an unsubstituted homocyclic or heterocyclic aryl radical or alkyl, alkoxy, alkylthio or halogen substituted homocyclic or heterocyclic aryl radical.

Illustratively, R³ and R⁴ can be phenyl, methoxyphenyl, ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl. In embodiments of the invention, for compounds having Formula I, R³ and R⁴ are independently phenyl or 4-methoxyphenyl.

Also, in embodiments of invention, for compounds having Formula I, n is from 8 to 10, or from 13 to 15, or from 18 to 20.

In an embodiment of the invention, in Formula I, R¹ and R² are both NH₂, as represented by Formula IV: 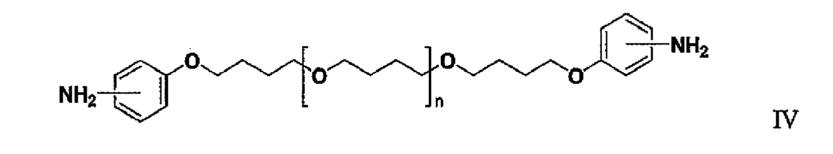 wherein n is from 0 to 30.

In embodiments of invention, for the compound having Formula IV, n is from 8 to 10, or from 13 to 15, or from 18 to 20.

In another embodiment of the invention, in Formula I, R¹ and R² are both NO₂, represented by Formula V: 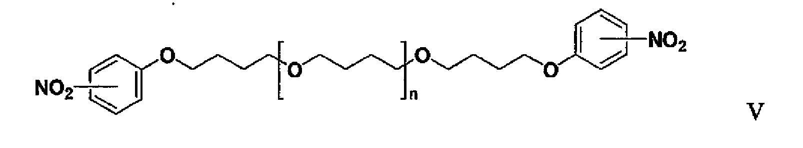 wherein n is from 0 to 30.

In embodiments of the invention, for the compound having Formula V, n is from 8 to 10, or from 13 to 15, or from 18 to 20.

In yet another embodiment of the invention, in Formula I, R¹ and R² are represented by Formulas II and III, respectively, representing a compound having Formula VI: 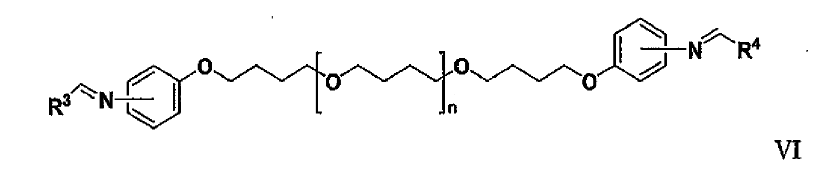 wherein R³ and R⁴ are as defined above, and n is from 0 to 30.

In embodiments of the invention, for the compound having Formula VI, n is from 8 to 10, or from 13 to 15, or from 18 to 20 and R³ and R⁴ are independently phenyl, methoxyphenyl (*e.g*., 4-methoxyphenyl), ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl.

Another aspect of the invention provides a compound having Formula VII: 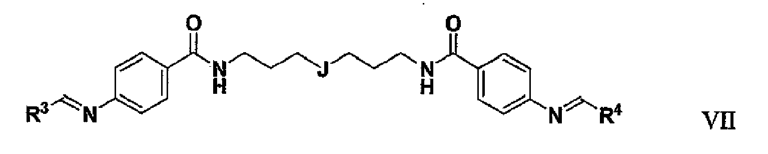 wherein R³and R⁴ are as defined above, and J is chosen from Formula VIII or Formula IX: 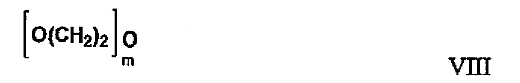 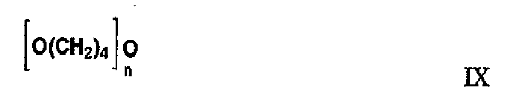 wherein in Formula VIII, m is from 1 to 16 and in Formula IX, n is from 1 to 30.

In embodiments of the invention, for the compound having Formula VII, substituent "m" in Formula VIII is from 8 to 10, or from 9 to 11, or from 13 to 15; substituent "n" in Formula IX is from 8 to 10, or from 13 to 15, or from 18-20; and R³ and R⁴ are independently phenyl, methoxyphenyl (*e*.*g*., 4-methoxyphenyl), ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl.

Yet another aspect of the invention provides a compound having Formula X: 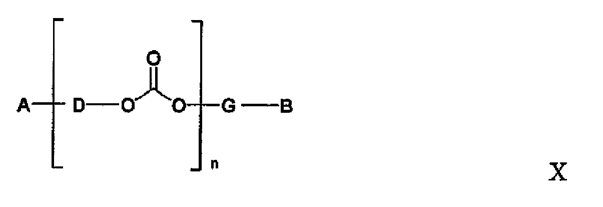 wherein n is from 1 to 15;
D and G are independently a C₂-C₁₈ alkylene, cycloalkyl, CH₂-cycloalkyl-CH₂, CH₂-aryl-CH₂, or CH₂-heteroaryl-CH₂; and
A and B are (1), (2), (3), or (4):

| | A | B |
|---|---|---|
| (1) | 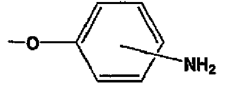 | 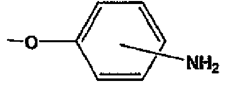 |
| (2) | 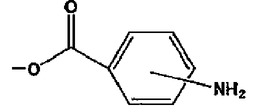 | 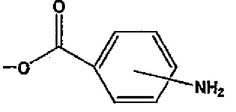 |
| (3) | 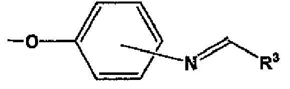 | 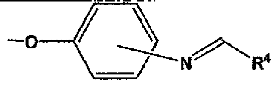 |
| (4) | 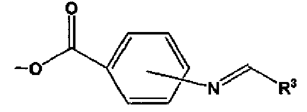 | 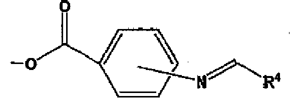 |

wherein R³ and R⁴ are as defined above.

In Formula X, above, when D or G is CH₂-aryl-CH₂ or CH₂-heteroaryl-CH₂, the aryl or heteroaryl can be further substituted.

Illustrative moieties for D and G in Formula X are hexyl; 1,4 dimethyl cyclohexyl; ortho, meta, or para xylyl; 2,5-furanyl; 2,5-thiophenyl; or a mixture thereof.

In embodiments of the invention, in Formula X, when A and B are (3) or (4) (as illustrated in Formulas XIII and XIV, below), R³ and R⁴ are independently phenyl, methoxyphenyl (*e*.*g*., 4-methoxyphenyl), ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl.

In embodiments of the invention, in Formula X, A and B are represented by a substituent of (1), (2), (3), or (4), so as to form compounds as represented by Formulas XI-XIV, respectively: 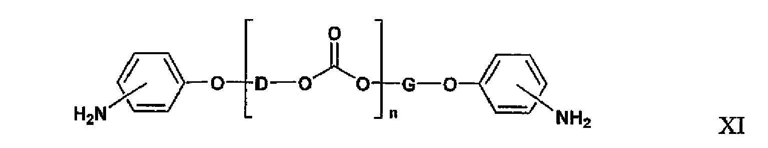 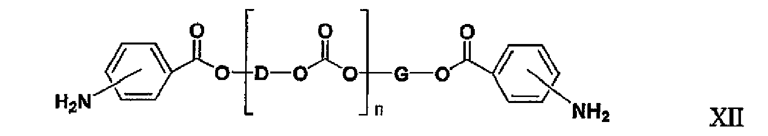 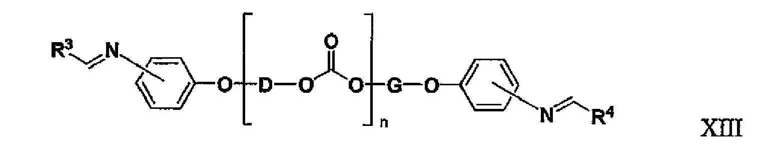 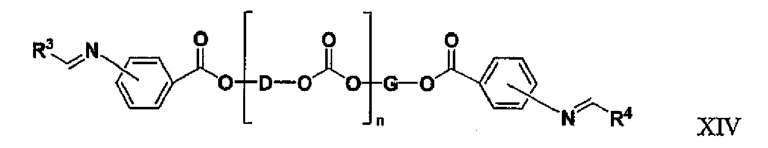 wherein the substituents are as defined above.

Still yet another aspect of the invention provides a compound having Formula XV: 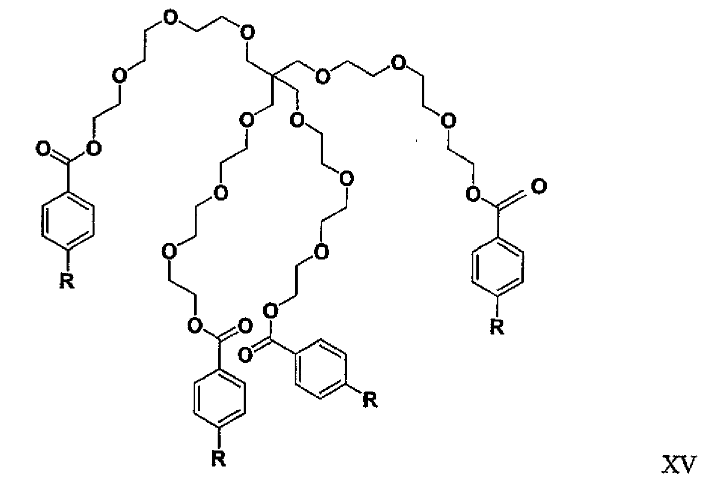 where each R is NH₂ (pentaerythritol-(4,3)-ethoxylate-tetrakis(4-aminobenzoate) or NO₂ (pentaerythritol-(4,3)-ethoxylate-tetrakis(4-nitrobenzoate).

A further aspect of the invention provides the compound 1,10-di(4-aminophenylcarbonyl)-1,4,7,10-tetraazadecane.

A still further aspect of the invention provides a compound having Formula XVI: 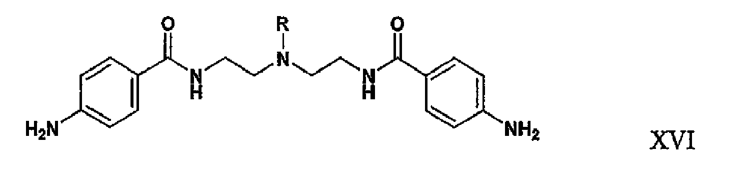 where R is H (1,7-di(4-aminophenylcarbonyl)-1,4,7-triazaheptane) or has Formula XVII (1,4,7-tri(4-aminophenylcarbonyl)-1,4,7-triazaheptane):

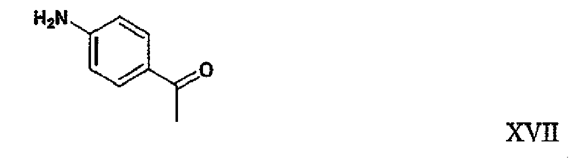

### Methods of Preparation of Compounds of the invention

Compounds of the present invention can be synthesized according to methods as described below.

Illustrative methods for making compounds having Formulas IV, V, and VI are as follows: A polytetramethylene glycol compound having Formula XVIII: 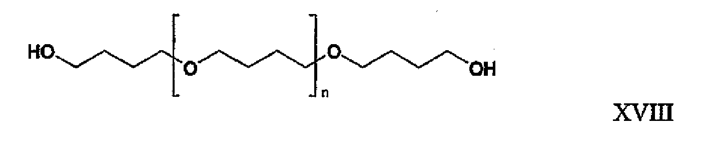 wherein n is from 0 to 30, is reacted with fluoro-nitrobenzene in the presence of NaH to form a compound having Formula V. Compounds having Formula XVIII are readily derived from simple diols as described in the literature. Illustrative compounds having Formula XVIII include TERATHANE® polytetramethylene ether glycols (INVISTA, Wichita, Kansas, USA), including those having molecular weight grades of 250, 650, 1000, 1400, and 1800.

The compound of Formula V is reduced, *e.g*. by use of a hydrogenation catalyst such as Ra-Ni, Pt, Zn/Acetic Acid, Pd, or a combination thereof, to form a compound having Formula IV.

The compound of Formula IV is reacted with one or more aldehydes having Formula XIX:

**R⁵CH=O** XIX

to form an imine having Formula VI.

In Formula XIX, R⁵ is an unsubstituted homocyclic or heterocyclic aryl radical or alkyl, alkoxy, alkylthio or halogen substituted homocyclic or heterocyclic aryl radical. For example, R⁵ can be phenyl, methoxyphenyl, ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl. Preferably, R⁵ is phenyl or 4-methoxyphenyl.

That is, examples of suitable aldehydes of Formula XIX include benzaldehyde, anisaldehyde (*e*.*g*., p-anisaldehyde), furfural, ethoxybenzaldehyde, butoxybenzaldehyde, hexyloxybenzaldehyde, octyloxybenzaldehyde, decyloxybenzaldehyde, dodecyloxybenzaldehyde, hexadecyloxybenzaldehyde, ethylbenzaldehyde, isopropylbenzaldehyde, dimethylbenzaldehyde, and pyridinecarboxaldehyde.

An imine compound having Formula VII can be made as follows: A compound having Formula XX: 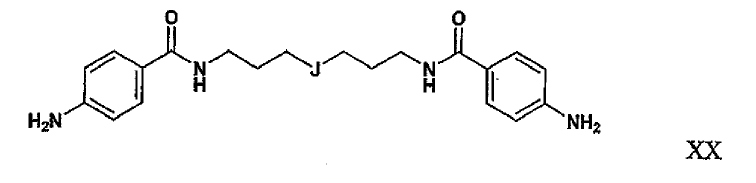 wherein J is chosen from Formula VIII or Formula IX: 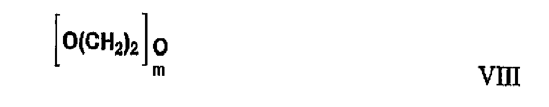 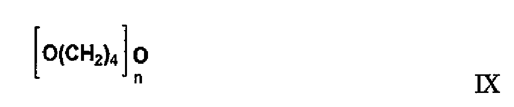 wherein in Formula VIII, m is from 1 to 16 and in Formula IX, n is from 1 to 30, is reacted with one or more aldehydes having Formula XIX as defined above, to form an imine having Formula VII.

Compounds having Formula XX can be made by converting bis(3-aminopropyl) terminated polyethylene glycol oligomers having the formula H₂N(CH₂)₃[O(CH₂)₂]ₘO(CH₂)₃NH₂, where m is from 1 to 16, or bis(3-aminopropyl) terminated polytetrahydrofuran oligomers having the formula H₂N(CH₂)₃[O(CH₂)₄]ₙO(CH₂)₃NH₂, where n is from 1 to 30, by mixing the oligomers with two moles of methyl 4-aminobenzoate in a lower alcohol (C₁₋₈) solvent (*e*.*g*., ethanol) and heating, and subsequently stripping the solvent. Alternatively, the oligomers can be reacted with 4-nitrobenzoyl chloride in pyridine, followed by reduction of the nitro-benzamides with hydrogen over PtO₂ in dimethylacetamide.

The bis(3-aminopropyl) terminated polyethylene glycol and polytetrahydrofuran oligomers described above can be made by condensing acrylonitrile with a polyethylene glycol or polytetrahydrofuran, respectively followed by reduction of the resulting compound. Bis(3-aminopropyl) terminated polytetrahydrofuran oligomers having formula H₂N(CH₂)₃[O(CH₂)₄]ₙO(CH₂)₃NH₂ are also available from Sigma-Aldrich having Mₙ of 350 or 1100.
Compounds having Formula XI can be made by reacting a polycarbonate diol having Formula XXI, below with nitrobenzoyl chloride (*e.g*., 2-, 3-, or 4-nitrobenzoyl chloride) in pyridine, followed by reduction of the nitrobenzoate with hydrogen over PtO₂ in dimethylacetamide.

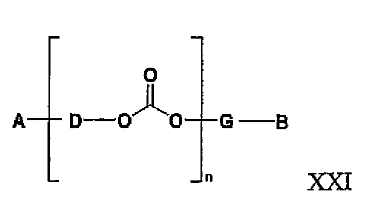

In Formula XXI, D and G are independently a C₂-C₁₈ alkylene, cycloalkyl, CH₂-cycloalkyl-CH₂, CH₂-aryl-CH₂, or CH₂-heteroaryl-CH₂, wherein the aryl or heteroaryl groups can be further substituted.

Illustrative compounds having Formula XXI include 1,6-hexanediol based-polycarbonate diol, 1,4-cyclohexane dimethanol and 1,6-hexanediol based-polycarbonatediol, and 1,4-cyclohexane dimethanol-based polycarbonatediol, which are available as UH-CARB, UM-CARB, and UC-CARB, respectively from UBE Industries, Ltd. (Tokyo, Japan).

Compounds having Formula XII, where the phenyl group is 3-amino or 4-amino substituted, can be prepared by mixing one mole of a polycarbonate diol of Formula XXI, above with two moles of a nitrobenzoyl chloride (*i*.*e*., 3-nitrobenzoyl chloride or 4-nitrobenzoyl chloride) and two moles of potassium carbonate in N-methyl pyrrolidinone (NMP) or N,N-dimethylacetamide (DMA) at 60 °C. The mixture is then filtered to remove inorganic salts and hydrogenated over Ra-Ni. A compound having Formula XII is isolated by separation upon the addition of four volumes of water.

The 2-aminobenzoate oligomers of Formula XII can be made by reacting a polycarbonate diol of Formula XXI with isatoic anhydride in the presence of NMP and potassium carbonate.

Imine compounds having Formula VI, VII, XIII, or XIV can be prepared by mixing a compound of Formula IV, XX, XI, or XII, respectively with a 10 to 25% molar excess of the desired aldehyde having Formula XIX and a catalytic amount of benzoic acid. The resulting stirred solution is heated to 50 to 70 °C and subjected to a vacuum of from 1 to 100 mbar. These conditions are held until all water evolved during the reaction is removed, typically 8 to 24 hours, to obtain the imine compounds.

The reactions to obtain imine compounds having Formulas VI, VII, XII, or XIV can be carried out with or without solvent. Suitable solvents include toluene, xylene, cyclohexane or heptane. After completion of the reaction, the solvent may be distilled from the reaction mixture to obtain the desired imine compound.

Pentaerythritol-(4,3)-ethoxylate-tetrakis(4-nitrobenzoate) can be made by reacting pentaerythritol ethoxylate (EO/OH) (3,4) (Sigma-Aldrich Chemical) with p-nitrobenzoyl chloride in the presence of pyridine. Pentaerythritol-(4,3)-ethoxylate-tetrakis(4-nitrobenzoate) can be reduced, *e*.*g*. by use of a hydrogenation catalyst such as Ra-Ni, Pt, Zn/Acetic Acid, Pd, or a combination thereof, to form pentaerythritol-(4,3)-ethoxylate-tetrakis(4-aminobenzoate), shown below:

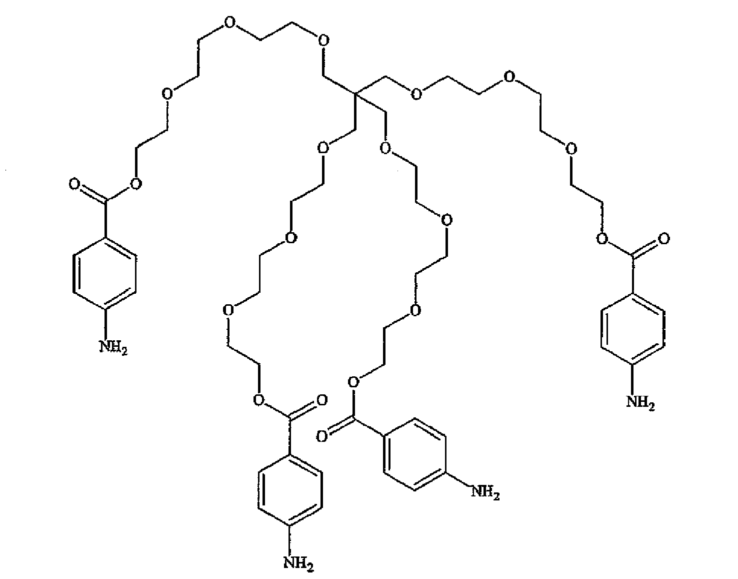

Pentaerythritol-(4,3)-ethoxylate-tetrakis(4-aminobenzoate) can alternatively be made by reacting pentaerythritol ethoxylate (EO/OH)(3,4) with p-aminobenzoic acid in the presence of 2-ethylhexanol and titanium (IV) isopropoxide, and distilling the excess 2-ethylhexanol to form pentaerythritol-(4,3)-ethoxylate-tetrakis(4-aminobenzoate).

The compound 1,10-di(4-aminophenylcarbonyl)-1,4,7,10-tetraazadecane can be made by mixing 1,4,7,10-tetraazdadecane (triethylenetetramine) (BASF, Florham Park, NJ, USA) with two moles of methyl 4-aminobenzoate in a lower alcohol (C₁₋₈) (*e*.*g*., ethanol) solvent, heating, and stripping the solvent, as shown in the scheme below.

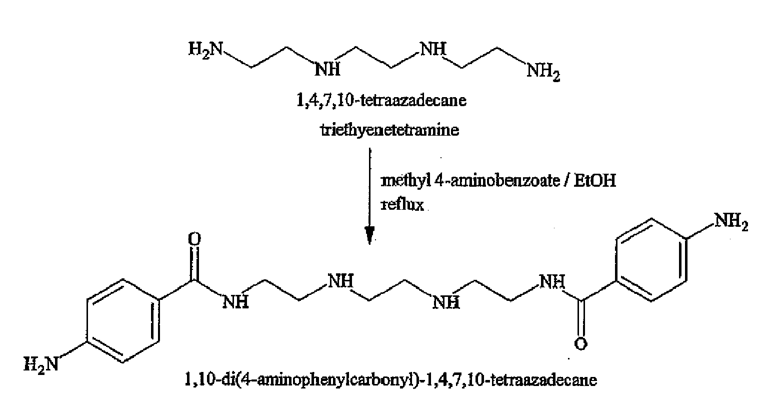

A compound having Formula XVI can be made by mixing in a lower alcohol (C₁₋₈) (*e*.*g*., methanol or ethanol) solvent 1,4,7-triazaheptane (diethylenetriamine) (BASF) and two or three moles of methyl 4-aminobenzoate, heating, and stripping the solvent. Depending upon the ratio of the above components, either 1,4,7-tri(4-aminophenylcarbonyl)-1,4,7-triazaheptane, 1,7-di(4-aminophenylcarbonyl)-1,4,7-triazaheptane, or both compounds are formed. A reaction scheme using methanol is provided below.

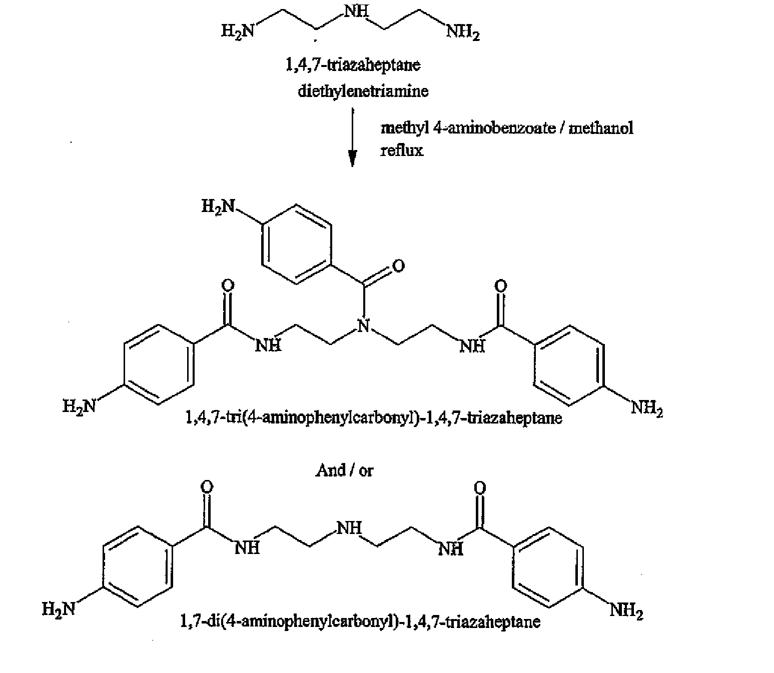

### Polyurea Compositions

The invention also provides polyurea compositions containing the novel compounds described above, cross-linked polyurea compositions, and UV-stabilized polyurea compositions.

The term "polyurea composition" encompasses compositions that are derived from the reaction product of an isocyanate component and a resin blend component containing amine-terminated polymer resins and/or amine-terminated chain extenders.

Generally, in polyurea compositions, the isocyanate can be aromatic or aliphatic in nature. The isocyanate can be monomer, polymer, or any variant reaction of isocyanates, quasi-prepolymer or a prepolymer. The prepolymer, or quasi-prepolymer, can be made of an amine-terminated polymer resin, or a hydroxyl-terminated polymer resin.

The resin blend component may contain amine-terminated polymer resins and/or amine-terminated chain extenders. Typically, the amine-terminated polymer resins will not have any intentional hydroxyl moieties. The resin blend may also contain additives, or non-primary components. These additives may contain hydroxyls, such as pre-dispersed pigments in a polyol carrier. Typically, the resin blend does not contain any catalyst.

In embodiments of the invention, the polyureas are "two-part" or two-component polyurea compositions. U.S. Pat. No. 5,162,481, incorporated by reference herein, is an illustrative disclosure of a two-part polyurea composition.

In one embodiment, polyureas of the invention comprise the reaction product of (a) one or more compounds of Formula IV, XI, or XII, (b) one or more aromatic polyisocyanates, polyurethane/urea prepolymers having aromatic isocyanate groups, or a combination thereof; and (c) a protic acid or a salt thereof, where the substituents have been defined above. These polyureas can be prepared by combining (a), (b), and (c) and curing by methods known in the art.

In embodiments of the invention, the two-part polyurea compositions contain about 75 to about 95% (*e*.*g*., about 78 to about 81%) by weight of one or more compounds of Formula IV, XI, or XII; about 5 to about 25% (*e.g*., about 15 to about 25%, or about 19 to about 22%) by weight of an aromatic polyisocyanate, a polyurethane/urea prepolymer having terminal aromatic isocyanate groups, or a combination thereof; and about 0.05 to about 5% by weight of a protic acid or a salt thereof.

In other embodiments of the invention, the polyureas are "one-part" polyurea compositions. By a "one-part" polyurea composition, it is meant that the polyurea may be cured (*i*.*e*. the polyurea is formed) by exposure to atmospheric moisture.

In contrast to "two-part" or two-component polyurea resins, a one-part composition does not require that separate components, such as organic polyisocyanate or modified or blocked polyisocyanate and polyamine and/or polyol be mixed at the time of application on site to effect curing. Rather, a one-part polyurea composition can be cured merely by exposure to moisture. A one-part polyurea composition prior to cure is referred to herein as a "prepolymer" or "prepolymer composition."

In embodiments of the invention, one-part polyurea prepolymer compositions comprise (a) one or more compounds having Formulas VI, VII, XIII, or XIV (referred to herein as the "imine compounds"), (b) one or more aromatic polyisocyanates, polyurethane/urea prepolymers having aromatic isocyanate groups, or a combination thereof; and (c) a protic acid or a salt thereof, where the substituents of Formulas VI, VII, XIII, and XIV are as defined above.

In embodiments of the invention, the one-part polyurea prepolymer compositions contain about 75 to about 95% by weight of the imine compound(s); about 5 to about 25% (*e*.*g*., about 15 to about 25%, or about 18 to about 22%) by weight of an aromatic polyisocyanate, a polyurethane/urea prepolymer having terminal aromatic isocyanate groups, or a combination thereof; and about 0.05 to about 5% by weight of a protic acid or a salt thereof

A one-part polyurea prepolymer composition of the present invention can be prepared by mixing under reduced pressure or in an inert atmosphere one or more of the imine compounds described above with aromatic polyisocyanates, polyurethane/urea prepolymers having terminal aromatic isocyanate groups, or a combination thereof; and a protic acid or a salt thereof.

Illustratively, an imine of the invention, including compounds having Formula VI, VII, XIII, or XIV, is heated in a container to a temperature of 45 to 55°C. An isocyanate component is heated to 55 to 80°C, added to the imine, and mixed well, to form a one-part polyurea prepolymer composition. The container is then sealed.

In forming the one-part polyurea prepolymer compositions and two-part polyurea compositions, the aromatic polyisocyanate can be an aromatic diisocyanate, carbodiimide modified polyisocyanate, biuret modified polyisocyanate, isocyanurate modified polyisocyanate, urethane modified polyisocyanate, or mixtures thereof In an embodiment, the aromatic diisocyanates are toluene diisocyanates or diphenylmethane diisocyanates including various mixtures of isomers thereof, such as ISONATE 143L and ISONATE 125M, both available from Dow Chemical Co., Midland, Mich., USA. ISONATE 125M, which is an MDI mixture of approximately 98% 4,4'-diphenylmethane diisocyanate and 2% 2,4'-diphenylmethane diisocyanate, is particularly preferred for use in the invention due to its relatively high purity level.

A polyurethane prepolymer having terminal aromatic isocyanate groups can be prepared by reacting an excess of aromatic polyisocyanate with polyol or polyamine, so that two or more free isocyanate groups remain in the resulting prepolymer.

The ratio of the number of amino groups in the amine formed by the hydrolysis of imine to the number of isocyanate groups contained in the polyisocyanate and/or the polyurethane/urea prepolymer having terminal aromatic isocyanate groups can be 0.5 to 2.0, *e.g*., 0.7 to 1.5.

Suitable protic acids for use in the polyurea and prepolymer compositions include carboxylic, sulfonic or phosphoric acids. Suitable carboxylic acids include aromatic carboxylic acids, such as benzoic acid, while examples of sulfonic acids are aromatic aliphatic sulfonic acids. In an embodiment, the amount of the protic acids can be in the range of from about 0.05 to about 5% by weight of the compositions.

The one-part polyurea prepolymer compositions of the invention can be diluted in a solvent directly prior to use (*i*.*e*., application and cure). In embodiments of the invention, inclusion of a solvent lowers the viscosity of the formulation and allows for better flow of the composition. The solvent that can be used is not particularly limited, so long as it does not react with the polyurea prepolymer composition. Suitable solvents include acetone, tetrahydrofuran, and 2-butanone.

Polyureas of the invention, including those of the reaction product of one or more compounds of Formula IV, XI, or XII; one or more aromatic polyisocyanates, polyurethane/urea prepolymers having aromatic isocyanate groups, or a combination thereof; and a protic acid or a salt thereof; and those formed by cure of polyurea prepolymers of one or more compounds having Formulas VI, VII, XIII, or XIV (referred to herein as the "imine compounds"); one or more aromatic polyisocyanates, polyurethane/urea prepolymers having aromatic isocyanate groups, or a combination thereof; and a protic acid or a salt thereof have enhanced properties compared to known polyurea compositions; including more consistent curing time and stability, and the ability to fine-tune hardness, flexibility, adhesion, resistance to chemical agents, and weather resistance.

It should be understood that the polyurea compositions and/or the polyurea prepolymer compositions of the invention can be combined with one another, or used as an additive for other compositions.

### Cross-linked Polyurea Compositions

Another aspect of the invention provides for cross-linked polyurea compositions, along with polyurea prepolymer compositions containing cross-linking agents.

Cross-linked polyureas of the invention include polyureas that are cross-linked with one or more of pentaerythritol-(4,3)-ethoxylate-tetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerythritol tetrakis(3-aminobenzoate), pentaerythritol tetrakis(4-aminobenzoate), or derivatives thereof.

The term "derivatives thereof" is intended encompass compounds that may be formed in situ when the cross-linking agent is combined with other components during the formation of a polyurea pre-polymer and/or a cross-linked polyurea.

Illustratively, cross-linked polyureas of the invention include those formed from (a) one or more compounds having Formula IV, XI, or XII, (b) one or more aromatic polyisocyanates, polyurethane/urea prepolymers having aromatic isocyanate groups, or a combination thereof, and (c) a protic acid or a salt thereof and are cross-linked with one or more of the cross-linking agents or derivatives thereof described above.

The invention also provides one-part polyurea prepolymer compositions which when cured form a cross-linked polyurea. For example, a one part polyurea prepolymer composition can contain one or more of pentaerythritol-(4,3)-ethoxylate-tetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, 1,7-di(4-aminophenylcarbonyl)-1,4,7-triazaheptane, Pentaerythritol tetrakis(2-aminobenzoate), pentaerythritol tetrakis(3-aminobenzoate), pentaerythritol tetrakis(4-aminobenzoate), or derivatives thereof.

For example, one-part polyurea prepolymer compositions that can contain one of the above compounds as a cross-linking agent include prepolymer compositions of (a) one or more compounds having Formula VI, Formula VII, Formula XIII, Formula XIV, or Formula XXII: 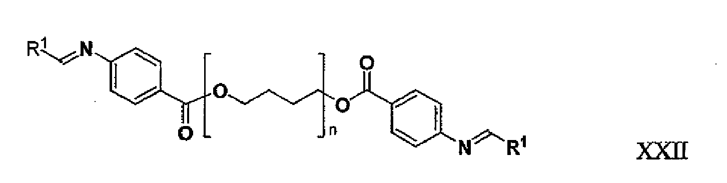 wherein in Formula XXII, n is from 3 to 30 and R¹ is an unsubstituted homocyclic or heterocyclic aryl radical or alkyl, alkoxy, alkylthio or halogen substituted homocyclic or heterocyclic aryl radical; (b) one or more aromatic polyisocyanates, polyurethane/urea prepolymers having aromatic isocyanate groups, or a combination thereof; and (c) a protic acid or a salt thereof.

In Formula XXII, R¹ can be phenyl, methoxyphenyl, ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, or pyridyl. Illustratively, in Formula XXII, R¹ is phenyl or 4 methoxyphenyl, and n is from 12 to 16.

A polyaldimine compound having Formula XXII can be prepared generally according to the methods described in PCT Publication No. WO 00/64860, U.S. Patent No. 6,552,155, International Application No. PCT/US2004/006654 filed March 4, 2005, and U.S. Pat. Appl. No. 10/793,644 filed March 5, 2004, all incorporated by reference herein in their entireties.

The amount of cross-linking agent present in the polyurea and polyurea prepolymer compositions is not particularly limited, so long as it provides suitable properties of strength and hardness while maintaining flexibility of the cured composition. For example, the cross-linking agent may be present in an amount ranging from about 0.1% and about 10% by weight of the polyurea and polyurea prepolymer compositions (*e*.*g*., from about 0.1 and about 5%, or about 1 and about 2%, most preferably about 1%).

Cross-linked polyureas of the invention can be formed by combining reaction products for forming a polyurea with the cross-linking agent and curing by methods known in the art. For example, to form a two-part cross-linked polyurea, the cross-linking agent can be added to and mixed with an amine component (*e*.*g*. a compound having Formula IV, XI, or XII); and the mixture is then mixed with an isocyanate component (*e*.*g*. a diphenylmethane diisocyanate (MDI)) and cured. In embodiments of the invention, the cross-linking agent is dissolved in a solvent, such as 2-butanone, prior to combining with the polyurea reaction products.

To form the inventive one-part cross-linking agent-containing polyurea prepolymer compositions, the cross-linking agent can be combined with an imine/polyaldimine compound; an aromatic polyisocyanate, a polyurethane/urea prepolymer having terminal aromatic isocyanate groups or a combination thereof; and a protic acid or a salt thereof, and stored in a moisture and air-proof container until ready for use. The cross-linking agent can also be added to the imine/polyaldimine and stored separately, and subsequently combined with other components.

Cross-linked polyureas of the invention provide tough and hard coatings for a variety of substrates, such as metal and non-metal (*e.g*. wood) surfaces without significant loss of flexibility compared to non-cross-linked polyureas.

Illustratively, polyureas cross-linked with pentaerythritol tetrakis(4-aminobenzoate) provide for particularly tough and abrasion resistant coatings that are particularly suitable for boat keels, as replacements for epoxy resins, in materials resistant to penetration, coatings for pipes transferring abrasive materials, and molded objects requiring strength and flexibility.

Polyureas cross-linked with pentaerythritol-(4,3)-ethoxylate-tetrakis(4-aminobenzoate) provide coatings which are particularly flexible, and thus are well suited for substrates having a high expansion coefficient, *e.g*. a porous clay surface for water proofing. Use of 1,10-di(4-aminophenylcarbonyl)-1,4,7,10-tetraazadecane, 1,4,7-tri(4-aminophenylcarbonyl)-1,4,7-triazaheptane, and 1,7-di(4-aminophenylcarbonyl)-1,4,7-triazaheptane, pentaerythritol tetrakis(2-aminobenzoate), and pentaerythritol tetrakis(3-aminobenzoate) as cross-linking agents in polyureas provide materials with enhanced strength with minimal or no loss of flexibility compared to non-cross-linked varieties.

Pentaerythritol-(4,3)-ethoxylate-tetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, and 1,7-di(4-aminophenylcarbonyl)-1,4,7-triazaheptane can be made as described above.

Pentaerythritol tetrakis(4-aminobenzoate) can be made according to the teachings of U.K. Patent No. 1,182,377, Gipp et al. (1970), incorporated by reference herein in its entirety. The compound has the following structure, formula, and molecular weight:

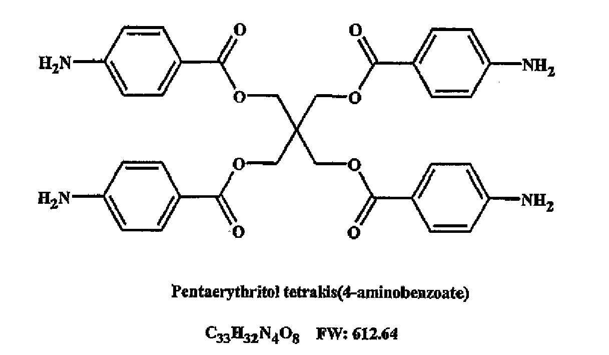

Pentaerythritol tetrakis(2-aminobenzoate) and pentaerythritol tetrakis(3-aminobenzoate) can be made according to the teachings of U.S. Patent No. 3,060,221, incorporated by reference herein in its entirety. Pentaerythritol tetrakis(2-aminobenzoate) can preferably be made by reacting pentaerythritol and isatoic anhydride in the presence of NMP and K₂CO₃, as shown in the scheme below:

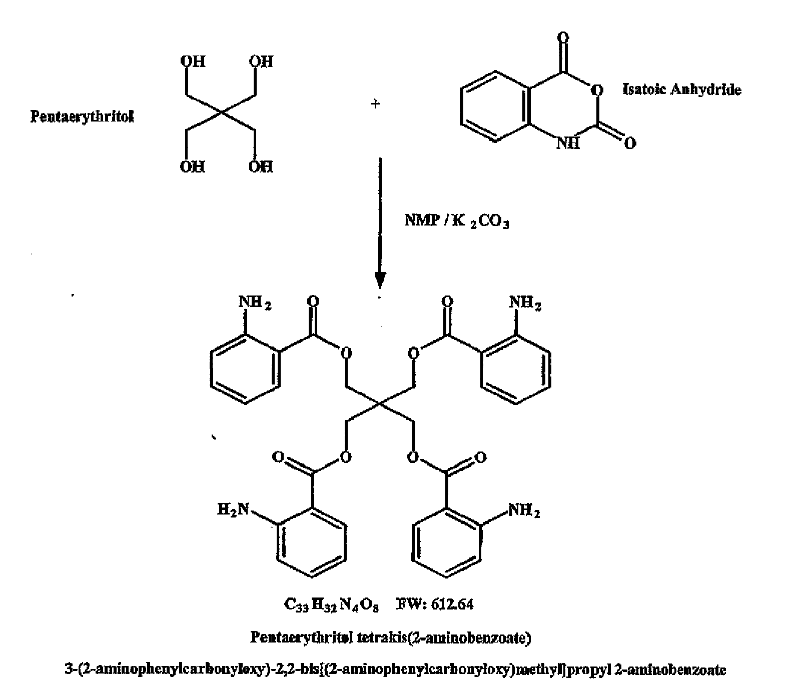

### UV-Stabilized Polyurea Compositions

Another aspect of the invention provides for novel UV-stabilized polyurea compositions comprising a polyurea and diphenyloxazole, along with polyurea prepolymer compositions containing diphenyloxazole.

In an embodiment of the invention, UV-stabilized polyureas containing diphenyloxazole include those formed from (a) one or more compounds having Formula IV, XI, or XII, (b) one or more aromatic polyisocyanates, polyurethane/urea prepolymers having aromatic isocyanate groups, or a combination thereof, and (c) a protic acid or a salt thereof.

The invention also provides one-part polyurea prepolymer compositions containing diphenyloxazole which when cured form a UV-stabilized polyurea. For example, one-part polyurea prepolymer compositions that can contain diphenyloxazole include prepolymer compositions of (a) one or more compounds having Formula VI, Formula VII, Formula XIII, Formula XIV, or Formula XXII, (b) one or more aromatic polyisocyanates, polyurethane/urea prepolymers having aromatic isocyanate groups, or a combination thereof; and (c) a protic acid or a salt thereof. Preferably, the diphenyloxazole is 2,5-diphenyloxazole.

In another embodiment of the invention, the cross-linked polyureas and prepolymer compositions containing a cross-linking agent further contain diphenyloxazole.

In the UV-stabilized polyurea compositions and prepolymers of the invention, diphenyloxazole is present in an amount effective to provide protection against UV degradation of the polyurea when cured. Preferably, diphenyloxazole is present in an amount ranging from 0.05 to 0.2% of the polyurea (or prepolymer) composition.

Diphenyloxazole can be obtained from a variety of sources, such as Sigma-Aldrich Chemical.

### Auxiliary Agents and Additives

In order to control viscosity, resin properties, and service life, auxiliary agents and/or additives such as fillers, thixotropic agents, plasticizers, adhesion improvers, metallic powders, inorganic or organic colorants, other stabilizers, biocides and solvents can be incorporated into the polyurea compositions and polyurea prepolymer compositions.

Examples of suitable fillers include calcium carbonate, talc, kaolin, aluminum sulfate, zeolite, diatomaceous earth, ground coconut shell, other natural products, polyvinylchloride paste resin, fumed silica, glass balloon and polyvinylidene chloride resin balloon. Examples of suitable thixotropic agents include colloidal silica, fatty acid amide wax, aluminum stearate, surface treated bentonite, polyethylene short fiber, Kevlar short fibers and phenol resin short fiber. Examples of suitable plasticizers include dioctyl phthalate, dibutyl phthalate, dilauryl phthalate, butyl benzyl phthalate, dioctyl adipate, diisodecyl adipate, diisodecyl phthalate and trioctyl phosphate. Illustrative adhesion improvers include known silane coupling agents.

Examples of metallic powder include metal flakes such as aluminum flakes, nickel flakes, stainless steel flakes, titanium flakes and bronze flakes. One type of metallic powder can be used individually, or a combination of two or more types can be used. The metallic powder can be blended in the range of preferably about 0.1 to about 20% by weight of the composition.

Examples of inorganic colorants include carbon black, graphite, molybdenum disulfide, titanium oxide, chromium oxide, iron oxide based colored pigments such as iron oxide red; and complex metal oxide based colored pigments such as composite inorganic oxide yellow and baked pigment.

Examples of organic colorants include phthalocyanine based colored pigments such as phthalocyanine green and phthalocyanine blue; perylene based colored pigments such as perylene red and perylene maroon; indanthrone based colored pigments such as indanthrone blue; azomethine based colored pigments such as azomethine yellow; benzimidazolone based colored pigments such as benzimidazolone yellow and benzimidazolone orange; quinacridone based colored pigments such as quinacridone orange, red, violet and quinacridone magenta; anthraquinone based colored pigments such as anthraquinone yellow and red; diketopyrolopyrrole based colored pigments such as diketopyrolopyrrole orange and red; isoindolinone based colored pigments such as isoindolinone yellow and orange; phthalimide based colored pigments such as phthalimide yellow; and dioxazine based colored pigments such as dioxazine violet.

Colorants can be blended in the range of about 0.1 to about 5% by weight of the composition. One type of colorant can be used individually, or a combination of two or more types can be used.

Sterically hindered amines, phenol compounds such as BHT, triazole compounds, organic sulfides, benzoxazoles, diphenyloxazole and other stabilizers including, for example the CHIMASSORB^{®} and TINUVIN^{®} product lines from Ciba Specialty Chemicals (Basel, Switzerland), can be added in the range of about 0.05 to about 2% by weight of the composition. Examples of suitable phenol and triazole compounds are illustrated in U.S. Patent Nos. 3,018,269 and 6,465,552, the patents incorporated by reference herein. As mentioned above, diphenyloxazole, particularly 2,5-diphenyloxazole, is particularly useful as a UV stabilizer and is an embodiment of the invention. When the additives have high moisture content, these additives must previously be dehydrated.

### Uses of the Polyureas of the Invention

The polyurea compositions of the invention provide for a variety of coating materials, including sealing materials, wall and roof covering materials, waterproofing materials, flooring materials, caulking materials, paints, and adhesives, as well as a fill for fiberglass and other fibrous and porous materials, depending upon the object for use.

The compositions of the invention can be applied to a substrate in any suitable manner, including (but not limited to) brushing, spraying, and soaking, and allowed to cure.

The polyurea and polyurea prepolymer compositions of the invention can be applied to a variety of substrates, such as metal and non-metal (*e*.*g*., wood or plastic) surfaces. Illustratively, the polyurea and polyurea prepolymer compositions can be applied to boat keels; wood (*e*.*g*. lumber), wood products (including for example materials made at least partially from wood or cellulose fiber), and wood structures (*e.g*. buildings, posts, shelters, marine pilings, support piers, wharfs, boat slips, bridges, ships, locks, and bank stabilizers) for weatherproofing or in accordance with the teachings of International Patent Application No. PCT/US2004/006654 filed March 4, 2005 to suppress degradation by wood borers (*e*.*g*., wood-boring insects such as termites, carpenter ants and wood-boring bees or marine borers such as shipworms or gribbles); or even to skis.

The polyurea compositions (including the cured prepolymers of the invention) are also useful as an undercoating for paint on metal or wood surfaces, as a coating for pipes in a corrosive environment as buried in the ground or underwater, and as an internal coating for pipes carrying corrosive or abrasive materials. Further, the smooth surface and resistance to adhesion of bio-fouling organisms makes the polyureas of the invention particularly suitable for marine applications.

The polyurea and polyurea prepolymer compositions can be applied to various substrates in an amount effective to provide a given property illustrated above. It should be understood by those skilled in the art the amount of a given composition would be required to be applied to provide, for example, a sealing or waterproofing property.

The present invention also encompasses the use of a container having a composition comprising any of the one-part polyurea prepolymer compositions described above, and instructions for its use.

### EXAMPLES

The following examples are provided to further illustrate the present invention. It is to be understood, however, that these examples are for purposes of illustration only and are not intended as a definition of the limits of the invention.

### EXAMPLE 1

A one-part polyurea prepolymer composition containing 720 g of polyaldimine, 180 g of an aromatic diisocyanate, and 12 g of benzoic acid was prepared as described below.

A polyaldimine having Formula XXII described above, where R¹ is 4-methoxyphenyl and j is from 12 to 16 was prepared generally following the teachings of U.S. Patent No. 6,552,155. Specifically, Versalink P-1000 (Air Products Corp.) was heated with an excess of 4-anisaldehyde in the presence of toluene and benzoic acid. The water generated in the reaction was removed, and the toluene was stripped from the resulting composition.

The polyaldimine and benzoic acid, and carbodiimide modified diphenyl diisocyanate Isonate M125 were heated separately to about 45°C and then mixed with high velocity stirring to form a one-part polyurea prepolymer composition.

### EXAMPLE 2

A cross-linking one-part polyurea prepolymer composition was prepared by the following method: A portion of the one-part polyurea prepolymer composition of Example 1 was mixed with pentaerythritol tetra-(4-aminobenzoate) to form a one-part polyurea prepolymer that cross-links upon cure. More specifically, to 900 grams of the one-part polyurea prepolymer of Example 1 was added 9 grams pentaerythritol tetra-(4-aminobenzoate), made according to the teachings of U.K. Patent No. 1,182,377. Prior to adding to the one-part polyurea prepolymer composition, the cross-linking agent was dissolved in 30 ml of 2-butanone at about 40-45°C. The resulting composition was mixed well and stored in an airtight container at room temperature.

As an excess of pure methylenediphenyl-diisocyanate Isonate M125 is believed to be present relative to the cross-linking agent, it is believed that the following derivative of the cross-linking agent forms in situ upon addition to the one-part polyurea, as shown below:

### Tetra-MDI adduct of the cross linking agent.

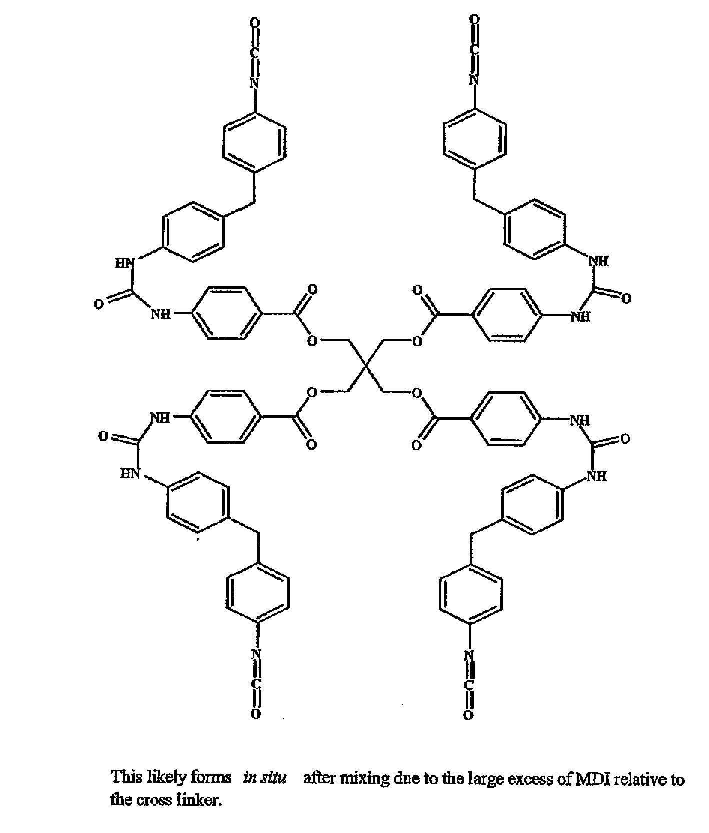

### EXAMPLE 3

Compositions prepared in accordance with Examples 1 and 2 (uncross-linked and cross-linked polyurea prepolymer, respectively) were tested for hardness after cure by applying to a microscope slide. Polyurea prepolymer compositions were prepared at cross-linking agent amounts of 0.5-1.5% by weight of the polyurea prepolymer. After curing for 24 hours at 21 °C and 25 to 45% relative humidity the cured compositions were marred with a stainless steel spatula. The resulting cured cross-linked polyureas were harder than the uncross-linked polyureas. Removing the cured polyureas from the slides demonstrated that all formulations were still flexible and would not break on repeated bending.

It is expected that at cross-linking agent compositions at or above 0.1% by weight of the polyurea prepolymer the resulting cured polymer will be harder than the un-cross-linked polyurea, with an optimal hardness level at cross-linking agent of between 1 and 2 weight %.

A polyurea prepolymer composition containing 1% cross-linking agent was applied to the keel of a 52 foot sailboat, the foundation of a newly constructed house, to cedar shakes and to various wood products. The formulations were found to exhibit toughness and durability as waterproofing agents and for protection for weathered and degraded wood products, as well as suitable as a fill for fiberglass and other fibrous and porous materials.

Addition of pentaerythritol tetra-(4-aminobenzoate) cross-linking agent did not negatively affect the shelf life of the resulting polyurea prepolymer of Example 2 compared to polyureas prepared in accordance with the teachings of U.S. Pat. No. 6,552,155, but improved the toughness and durability of the cured polymer.

It will be apparent to those skilled in the relevant art that the disclosed invention may be modified in numerous ways and may assume embodiments other than the forms specifically set out and described above. Accordingly, it is intended by the appended claims to cover all modifications of the invention which fall within the true spirit and scope of the invention.

All patents and publications cited herein, along with U.S. Provisional Application No. 60/570,897 for which this application claims priority, are fully incorporated by reference herein in their entireties.

Also, as used herein in accordance with all of the embodiments of the invention, the term "a" is intended to include its ordinary meaning of "one or more" unless specifically stated to the contrary.

The present application also discloses the following clauses:
1. A compound having Formula I: 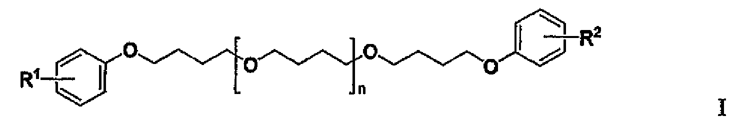 wherein in Formula I, n is from 0 to 30;
R¹ and R² are independently NH₂ or NO₂, or R¹ and R² are represented by Formulas II and III, respectively: 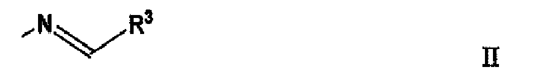 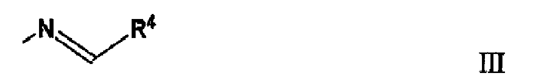 wherein in Formulas II and III, R³ and R⁴ are independently an unsubstituted homocyclic or heterocyclic aryl radical or alkyl, alkoxy, alkylthio or halogen substituted homocyclic or heterocyclic aryl radical.
2. The compound of clause 1, having Formula IV: 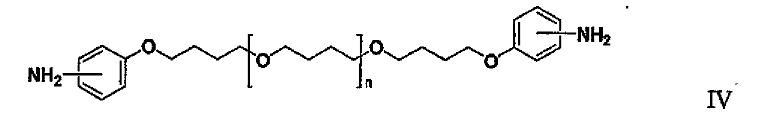 wherein in Formula IV, n is from 0 to 30.
3. The compound of clause 1, having Formula V: 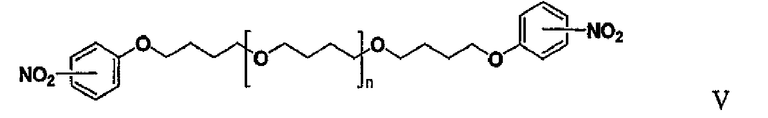 wherein in Formula V, n is from 0 to 30.
4. The compound of clause 1, having Formula VI: 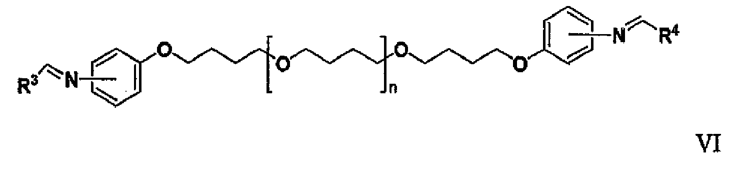 wherein in Formula VI, n is from 0 to 30, and R³ and R⁴ are independently an unsubstituted homocyclic or heterocyclic aryl radical or alkyl, alkoxy, alkylthio or halogen substituted homocyclic or heterocyclic aryl radical.
5. A compound having Formula VII: 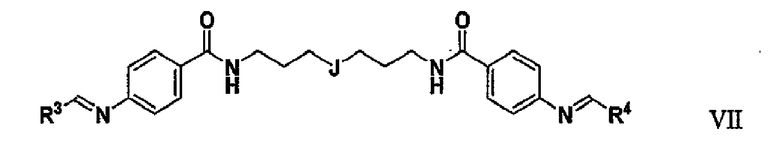 wherein J is chosen from Formula VIII or Formula IX: 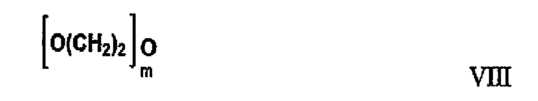 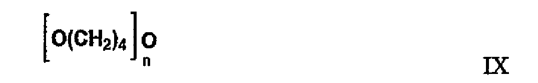 wherein in Formula VII, R³ and R⁴ are independently an unsubstituted homocyclic or heterocyclic aryl radical or alkyl, alkoxy, alkylthio or halogen substituted homocyclic or heterocyclic aryl radical; in Formula VIII, m is from 1 to 16; and in Formula IX, n is from 1 to 30.
6. The compound of clause 4 or 5, wherein R³ and R⁴ are independently selected from the group consisting of phenyl, methoxyphenyl, ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, and pyridyl.
7. A compound having Formula X: 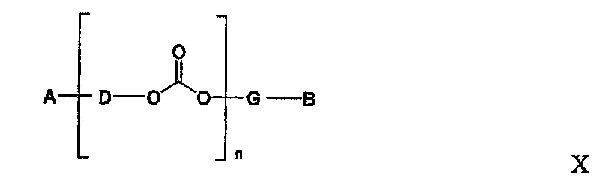 wherein n is from 1 to 15;
D and G are independently a C₂-C₁₈ alkylene, cycloalkyl, CH₂-cycloalkyl-CH₂, CH₂-aryl-CH₂, or CH₂-heteroaryl-CH₂; and A and B are one of (1), (2), (3), or (4):

| | A | B |
|---|---|---|
| (1) | 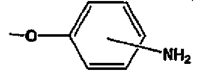 | 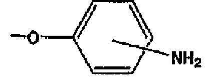 |
| (2) | 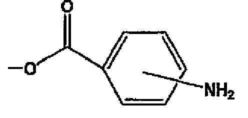 | 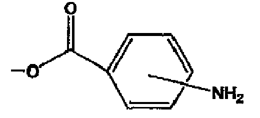 |
| (3) | 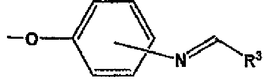 | 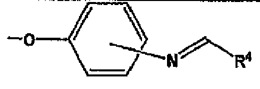 |
| (4) | 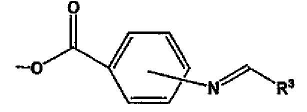 | 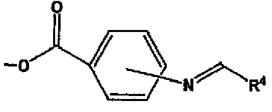 |

wherein in (3) and (4), R³ and R⁴ are independently an unsubstituted homocyclic or heterocyclic aryl radical or alkyl, alkoxy, alkylthio or halogen substituted homocyclic or heterocyclic aryl radical.
8. The compound of clause 7, having Formula XI or Formula XII: 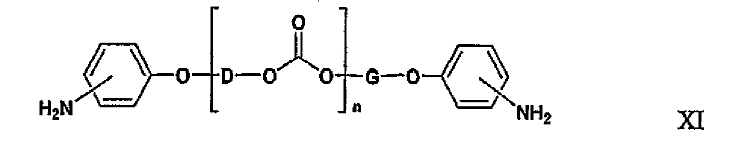 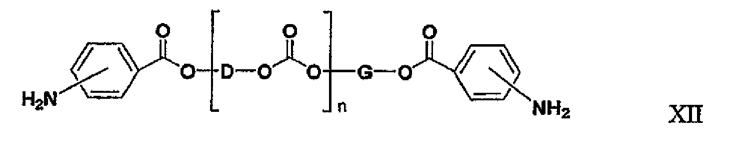 wherein in Formula XI or Formula XII,
n is from 1 to 15; and
D and G are independently a C₂-C₁₈ alkylene, cycloalkyl, CH₂-cycloalkyl-CH₂, CH₂-aryl-CH₂, or CH₂-heteroaryl-CH₂.
9. The compound of clause 8, having Formula XI: 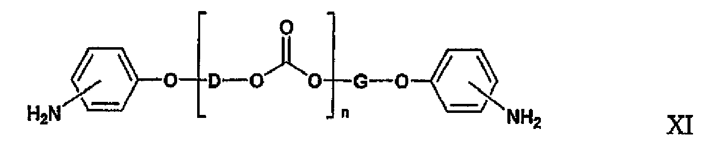 wherein in Formula XI,
n is from 1 to 15; and
D and G are independently a C₂-C₁₈ alkylene, cycloalkyl, CH₂-cycloalkyl-CH₂, CH₂-aryl-CH₂, or CH₂-heteroaryl-CH₂.
10. The compound of clause 8, having Formula XII: 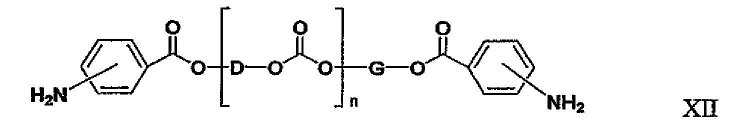 wherein in Formula XII,
n is from 1 to 15; and
D and G are independently a C₂-C₁₈ alkylene, cycloalkyl, CH₂-cycloakyl-CH₂, CH₂-aryl-CH₂, or CH₂-heteroaryl-CH₂.
11. The compound of clause 10, wherein the NH₂ groups are 3- or 4-substituted on the phenyl groups.
12. The compound of clause 10, wherein the NH₂ groups are 2- substituted on the phenyl groups.
13. The compound of clause 7, having Formula XM or Formula XIV: 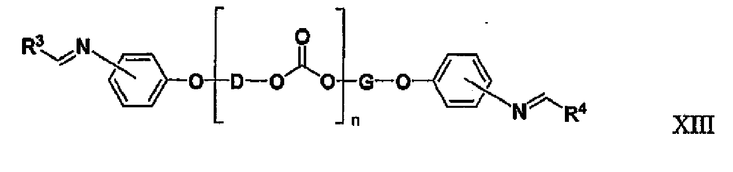 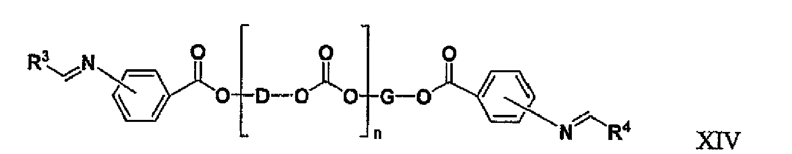 wherein in Formulas XIII and XIV,
n is from 1 to 15;
D and G are independently a C₂-C₁₈ alkylene, cycloalkyl, CH₂-cycloalkyl-CH₂, CH₂-aryl-CH₂, or CH₂-hcteroayl-CH₂; and R³ and R⁴ are independently an unsubstituted homocyclic or heterocyclic aryl radical or alkyl, alkoxy, alkylthio or halogen substituted homocyclic or heterocyclic aryl radical.
14. The compound of clause 13, having Formula XIII: 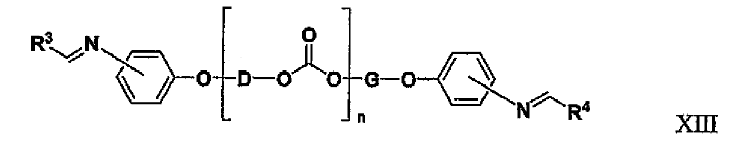 wherein in Formula XIII,
n is from 1 to 1.5;
D and G are independently a C₂-C₁₈ alkylene, cycloalkyl, CH₂-cycloalkyl-CH₂, CH₂-aryl-CH₂, or CH₂-hcteroaryl-CH₂; and R³ and R⁴ are independently an unsubstituted homocyclic or heterocyclic aryl radical or alkyl, alkoxy, alkylthio or halogen substituted homocyclic or heterocyclic aryl radical.
15. The compound of clause 13, having Formula XIV: 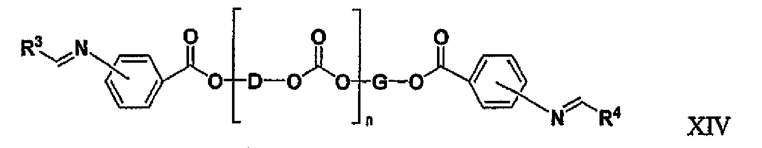 wherein in Formula XTV,
n is from 1 to 15;
D and G are independently a C₂-C₁₈ akylene, cycloalkyl, CH₂-cycloalkyl-CH₂, CH₂-aryl-CH₂, or CH₂-heteromyl-CH₂; and
R³ and R⁴ are independently an unsubstituted homocyclic or heterocyclic aryl radical or alkyl, alkoxy, alkylthio or halogen substituted homocyclic or heterocyclic aryl radical.
16. The compound of any one of clause 7 and 13 to 15, wherein D and G are independently hexyl, 1,4 dimethyl cyclohexyl, ortho, meta, or para xylyl, 2,5-furanyl, 2,5-thiophenyl, or a mixture thereof; and R³ and R⁴ are independently selected from the group consisting of phenyl, methoxyphenyl ethoxyphenyl, butoxyphenyl, hexyloxyphenyl, octyloxyphenyl, decyloxyphenyl, dodecyloxyphenyl, hexadecyloxyphenyl, ethylphenyl, isopropylphenyl, dimethylphenyl, furyl, and pyridyl.
17. A compound having Formula XV: 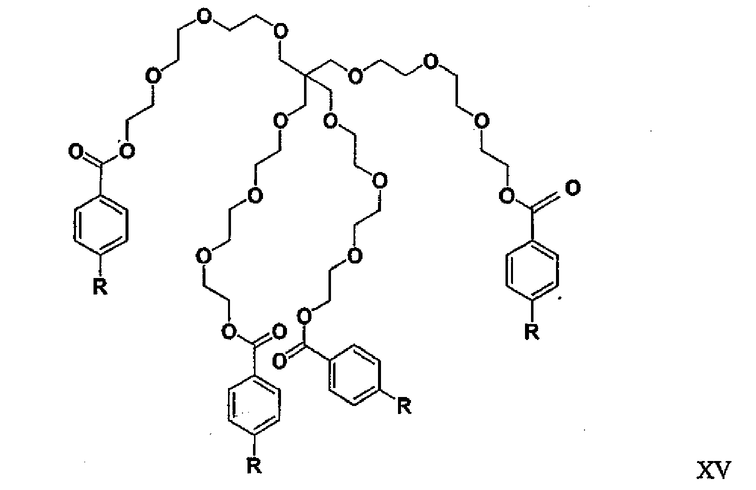 wherein each R is independently NO₂ or NH₂.
18. The compound of clause 17, wherein each R is NH₂.
19. The compound of clause 17, wherein each R is NO₂.
20. 1,10-di(4-aminophenylcarbonyl)-1,4,7,10-tetraadecane.
21. A compound having Formula XVI: 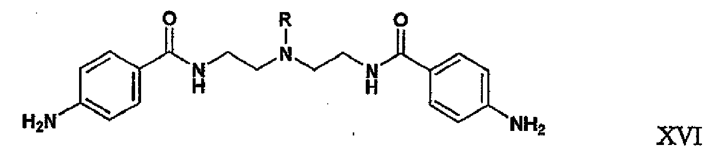 wherein R is H or has Formula XVII: 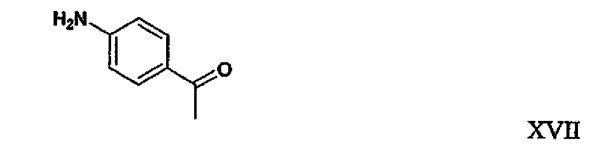 22. A method of making a compound of clause 18, comprising reducing pentaerythritol-(4,3)-ethoxylate-tetrakis(4-nitrobenzoate) to form the compound of clause 18.
23. A method of making a compound of clause 19, comprising reacting pentaerythritol ethoxylate (EO/OH) (3,4) with p-nitrobenzoyl chloride in the presence of pyridine, to form the compound of clause 19.
24. A method of making a compound of clause 18, comprising reacting pentaerythritol ethoxylate (EO/OH) (3,4) with p-aminobenzoic acid in the presence of 2-ethylhexanol and titanium (IV) isopropoxide, and distilling the excess 2-ethylhexanol to form the compound of clause 18.
25. A polyurea cross-linked with one or more of pentaerythritol-(4,3)-ethoxylate-tetrakis(4-aminabenzoate), 1,10-di(4-aminophenylcairbonyl)-1,4,7,10-tetraazadecane, 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerytritol tetrakis(3-aminobenzoate), pentaerythritol tetrakis(4-aminobenzoate), or derivatives thereof.
26. A one-part polyurea prepolymer composition, comprising:
(a) one or more compounds having Formula XXII: 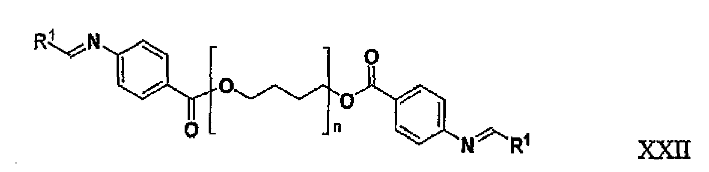 wherein in Formula XXII, n is from 3 to 30 and R1 is an unsubstituted homocyclic or heterocyclic aryl radical or alkyl, alkoxy, alkylthio or halogen substituted homocyclic or heterocyclic aryl radical;
(b) one or more aromatic polyisocyanates, polyurethane/urea prepolymers having aromatic isocyanate groups, or a combination thereof;
(c) a protic acid or a salt thereof; and
(d) a cross-linking agent comprising one or more of pentaerythritol-(4,3)-ethoxylate-tetrakis(4-aminobenzoate), 1,10-di(4-aminophenylcarbonyl)-1,4,7,10-tetraazadecane), 1,4,7-tri(4-aminophenylcarbonyl)-1,4,7-briazahetane, 1,7-di(4-aminophenylearbonyl)-1,4,7-triazaheptane, pentaerythritol tetrakis(2-aminobenzoate), pentaerythritol tetrakis(3-aminobenzoate), pentaerythritol tetrakis(4-aminobenzoate), or derivatives thereof.
27. A cross-linked polyurea comprising the reaction product of the composition of clause 26 when cured.
28. A method of making a cross-linked polyurea, comprising combining polyurea reaction products with a cross-linking agent comprising one or more of pentaerythritol-(4,3)-ethoxylate-tetrakiss(4-aminobenzoate), 1,10-di(4-aminophenylcarbonyl)-1,4,7,10-tetraazadecane), 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerythritol tetrakis(3-aminobenzoate), and pentaerythritol tetrakis(4-aminobenzoate), and curing to form the cross-linked polyurea.
29. A method of making the one-part polyurea prepolymer composition of clause 26, comprising combining:
(a) one or more compounds having Formula XXII: 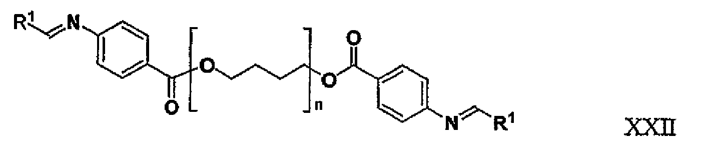 wherein in Formula XXII, n is from 3 to 30 and R¹ is an unsubstituted homocyclic or heterocyclic aryl radical or alkyl, alkoxy, alkylthio or halogen substituted homocyclic or heterocyclic aryl radical;
(b) one or more aromatic polyisocyanates, polyurethane/urea prepolymers having aromatic isocyanate groups, or a combination thereof;
(c) a protic acid or a salt thereof; and
(d) a cross-linking agent comprising one or more of pentaerythritol-(4,3)-ethoxylate-tetrakis(4-aminobenzoate), 1,10-di(4-aminophenylcarbonyl)-1,4,7,10-tetraazadecane, 1,4,7-tri(4-aminophenylearbonyl)-1,4,7-triazaheptane, 1,7-di(4-aminophenylcarbonyl)-1,4,7-triazaheptane, pentaerythritol tetrakis(2-aminobenzoate), pentaerythritol tetrakis(3-aminobenzoate), and pentaerythritol tetrakis(4-aminobenzoate) to form the one-part polyurea prepolymer composition of clause 26.
30. A method of making a cross-linked polyurea composition, comprising curing the one-part polyurea prepolymer composition of clause 26.
31. An article of manufacture comprising a substrate having the cross-linked polyurea of clause 25 applied thereto.
32. An article of manufacture comprising a substrate having the cross-linked polyurea of clause 27 applied thereto.
33. A method of making a compound of clause 2, comprising reducing a compound of clause 3 to form the compound of clause 2.
34. A method of making a compound of clause 3, comprising reacting a polytetramethylene ether glycol having Formula XVIII: 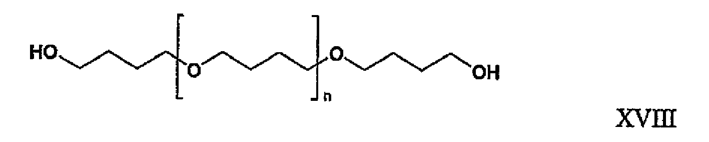 with fluoro-nitrobenzene in the presence of NaH to form the compound of clause 3; wherein in Formula XVIII, n is from 0 to 30.
35. A method for making a compound of clause 4, comprising reacting a compound of clause 2 with one or more aldehydes having Formula XIX

**R⁵CH=O** XIX

wherein in Formula XII, R⁵ is an unsubstituted homocyclic or heterocyclic aryl radical or alkyl, alkoxy, alkylthio or halogen substituted homocyclic or heterocyclic aryl radical.
36. A one-part polyurea prepolymer composition, comprising:
(a) one or more compounds of clause 4;
(b) one or more aromatic polyisocyanates, polyurethane/urea prepolymers having aromatic isocyanate groups, or a combination thereof; and
(c) a protic acid or a salt thereof
37. A polyurea comprising the reaction product of the composition of clause 36 when cured.
38. A method of making the one-part polyurea prepolymer composition of clause 36, comprising combining
(a) one or more compounds of clause 4;
(b) one or more aromatic polyisocyanates, polyurethane/urea prepolymers having aromatic isocyanate groups, or a combination thereof; and
(c) a protic acid or a salt thereof; to form the one-part polyurea prepolymer composition of clause 36.
39. A method of making a one-part polyurea composition, comprising curing the one-part polyurea prepolymer composition of clause 36.
40. An article of manufacture comprising a substrate having the polyurea of clause 37 applied thereto.
41. A polyurea comprising the reaction product of:
(a) one or more compounds of clause 2;
(b) one or more aromatic polyisocyanates, polyurethane/urea prepolymers having aromatic isocyanate groups, or a combination thereof; and
(c) a protic acid or a salt thereof.
42. A method of making the polyurea of clause 41, comprising combining
(a) one or more compounds of clause 2;
(b) one or more aromatic polyisocyanates, polyurethane/urea prepolymers having aromatic isocyanate groups, or a combination thereof; and
(c) a protic acid or a salt thereof; and curing to form the polyurea of clause 41.
43. An article of manufacture comprising a substrate having the polyurea of clause 41 applied thereto.
44. A method for making a compound of clause 5, comprising reacting a compound having Formula XX: 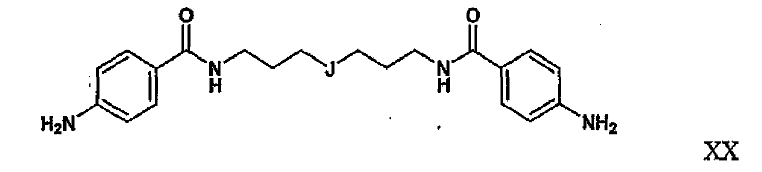 with one or more aldehydes having Formula XIX:

**R⁵CH=O** XIX

to form the compound of clause 5;
wherein in Formula XX, J is chosen from Formula VIII or Formula IX: 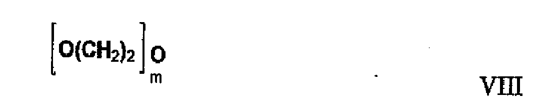 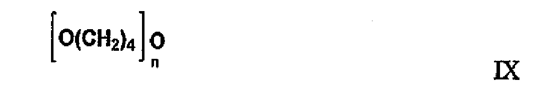 wherein in Formula VIII, m is from 1 to 16 and in Formula IX, n is from 1 to 30, and
wherein in Formula XIX, R⁵ is an unsubstituted homocyclic or heterocyclic aryl radical or alkyl, alkoxy, alkylthio or halogen substituted homocyclic or heterocyclic aryl radical.
45. A one-part polyurea prepolymer composition comprising:
(a) one or more compounds of clause 5;
(b) one or more aromatic polyisocyanates, polyurethane/urea prepolymers having aromatic isocyanate groups, or a combination thereof; and
(c) a protic acid or a salt thereof.
46. A polyurea comprising the reaction product of the composition of clause 45 when cured.
47. A method of making the one-part polyurea prepolymer composition of clause 45, comprising combining:
(a) one or more compounds of clause 5;
(b) one or more aromatic polyisocyanates, polyurethane/urea prepolymers having aromatic isocyanate groups, or a combination thereof; and
(c) a protic acid or a salt thereof, to form the one-part polyurea prepolymer composition of clause 45.
48. A method of making a one-part polyurea composition, comprising curing the one-part polyurea prepolymer composition of clause 45.
49. An article of manufacture comprising a substrate having the polyurea of clause 46 applied thereto.
50. A method of making the compound of clause 9, comprising reacting a compound having Formula XXI: 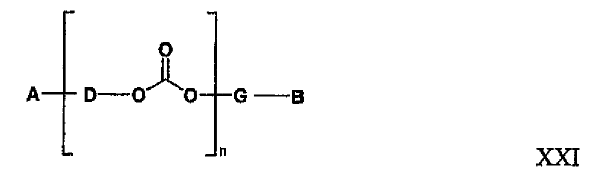 with nitrobenzoyl chloride in pyridine; and reducing to form the compound of clause 9;
wherein in Formula XXI,
n is from 1 to 15; and
D and G are independently a C₂-C₁₈ alkylene, cycloalkyl, CH₂-cyloakyl-CH₂, CH₂-aryl-CH₂, or CH₂-heteroaryl-CH₂.
51. A method of making the compound of clause 11, comprising reacting a compound having Formula XXI: 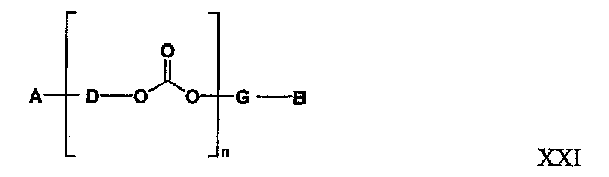 with nitrobenzoyl chloride and potassium carbonate in N-methyl pyrrolidinone or N,N-dimethylbenzenamine and reducing to form the compound of clause 11;
wherein in Formula XXI,
n is from 1 to 15; and
D and G are independently a C₂-C₁₈ alkylene, cycloalkyl, CH₂-cycloalkyl-CH₂, CH₂-aryl-CH₂, or CH₂-heteroaryl-CH₂.
52. A method of making the compound of clause 12, comprising reacting a compound having Formula XXI: 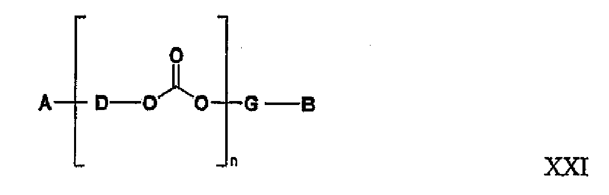 with isatoic anhydride in the presence of N-methyl pyrrolidinone and potassium carbonate to form the compound of clause 12;
wherein in Formula XXI,
n is from 1 to 15; and
D and G are independently a C₂-C₁₈ alkylene, cycloalkyl, CH₂-cycloalkyl-CH₂, CH₂-aryl-CH₂, or CH₂-heteroaryl-CH₂.
53. A method of making the compound of clause 14, comprising reacting a compound of clause 9 with one or more aldehydes having Formula XIX:

**R⁵CH=O** XIX

wherein in Formula XIX, R⁵ is an unsubstituted homocyclic or heterocyclic aryl radical or alkyl, alkoxy, alkylthio or halogen substituted homocyclic or heterocyclic aryl radical.
54. A method of making the compound of clause 15, comprising reacting the compound of clause 10 with one or more aldehydes having Formula

**R⁵CH=O** XIX

wherein in Formula XIX, R⁵ is an unsubstituted homocyclic or heterocyclic aryl radical or alkyl, alkoxy, alkylthio or halogen substituted homocyclic or heterocyclic aryl radical.
55. A one-part polyurea prepolymer composition, comprising:
(a) one or more compounds of clause 13;
(b) one or more aromatic polyisocyanates, polyurethane/urea prepolymers having aromatic isocyanate groups, or a combination thereof; and
(c) a protic acid or a salt thereof.
56. A polyurea comprising the reaction product of the composition of clause 55 when cured.
57. A method of making the one-part polyurea prepolymer composition of clause 55, comprising combining
(a) one or more compounds of clause 13;
(b) one or more aromatic polyisocyanates, polyurethane/urea prepolymers having aromatic isocyanate groups, or a combination thereof; and
(c) a protic acid or a salt thereof, to form the one-part polyurea prepolymer composition of clause 55.
58. A method of making a one-part polyurea composition, comprising curing the one-part polyurea prepolymer composition of clause 55.
59. An article of manufacture comprising a substrate having the polyurea of clause 56 applied thereto.
60. A polyurea comprising the reaction product of:
(a) one or more compounds of clause 8;
(b) one or more aromatic polyisocyanates, polyurethane/urea prepolymers having aromatic isocyanate groups, or a combination thereof; and
(c) a protic acid or a salt thereof.
61. A method of making the polyurea of clause 60, comprising combining
(a) one or more compounds of clause 8;
(b) one or more aromatic polyisocyanates, polyurethane/urea prepolymers having aromatic isocyanate groups, or a combination thereof; and
(c) a protic acid or a salt thereof; and curing to form the polyurea of clause 60.
62. An article of manufacture comprising a substrate having the polyurea of clause 60 applied thereto.
63. A method of making the compound of clause 20, comprising reacting triethylenetetramine with methyl 4-aminobenzoate in the presence of a lower alcohol (C₁₋₈) solvent to form the compound of clause 20.
64. A method of making the compound of clause 21, comprising reacting dietheylenetriamine with methyl 4-aminobenzoate in the presence of a lower alcohol (C₁₋₈) solvent to form the compound of clause 21.
65. The one-part polyurea prepolymer composition of any one of clause 36, 45, and 55 further comprising a cross-linking agent comprising one or more of pentaerythritol-(4,3)-ethoxylate-tetrakis(4-aminobenzoate), 1,10-di(4-aminophenylcarbonyl)-1,4,7,10-tetraazadecane), 1,4,7-tri(4-aminophenylearbonyl)-1,4,7-triazaheptane, 1,7-di(4-aminophenylearbonyl)-1,4,7-triazaheptane, pentaerythritol tetrakis(2-aminobenzoate), pentaerythritol tetrakis(3-aminobenzoate), pentaerythritol tetrakis(4-aminobenzoate), or derivatives thereof.
66. The polyurea of any one of clause 37, 41, 46, 56, and 60 further comprising a cross-linking agent comprising one or more of pentaerythritol-(4,3)-ethoxylate-tetrakis(4-aminobenzoate), 1,10-di(4-aminophenylcarbonyl)-1,4,7,10-tetraazadecane), 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerythriol tetrakis(3-aminobenzoate), pentaerythritol tetrakis(4-aminobenzoate), or derivatives thereof.
67. A UV-stabilized polyurea, comprising a polyurea and a diphenyloxazole in an amount effective to provide protection against UV degradation of the polyurea.
68. A one-part polyurea prepolymer composition, comprising:
(a) one or more compounds having Formula XXII: 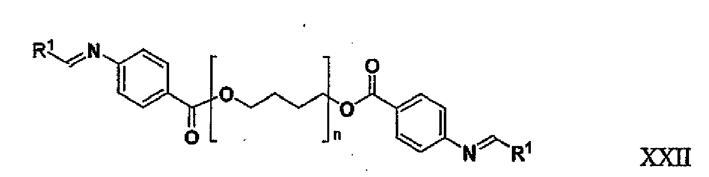 wherein in Formula XXII, n is from 3, to 30 and R¹ is an unsubstituted homocyclic or heterocyclic aryl radical or alkyl, alkoxy, alkylthio or halogen substituted homocyclic or heterocyclic aryl radical;
(b) one or more aromatic polyisocyanates, polyurethane/urea prepolymers having aromatic isocyanate groups, or a combination thereof;
(c) a protic acid or a salt thereof; and
(d) a diphenyloxazole in an amount effective to provide protection against UV degradation of the one-part polyurea prepolymer composition when cured.
69. The one-part polyurea prepolymer composition of any one of clause 26, 36, 45, and 55 further comprising a diphenyloxazole in an amount effective to provide protection against UV degradation when cured.
70. The polyurea of any one of clause 25, 27, 37, 41, 46, 56, and 60 further comprising diphenyloxazole in an amount effective to provide protection against UV degradation.
71. An article of manufacture comprising a substrate having the reaction product of the composition of clause 68 or clause 69 applied thereto.
72. An article of manufacture comprising a substrate having the polyurea of clause 70 applied thereto.

## Claims

1. A compound having Formula XV: 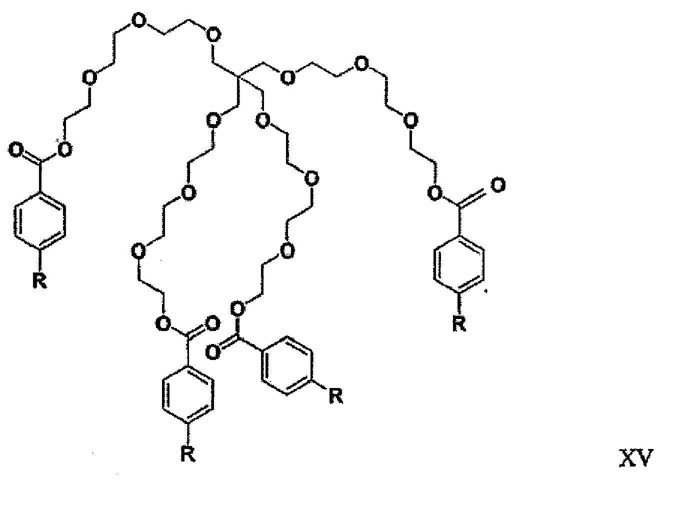 wherein each R is independently NO₂ or NH₂.

2. The compound of claim 1, wherein each R is NH₂.

3. The compound of claim 1, wherein each R is NO₂.

4. 1,10-di(4-aminophenylcarbonyl)-1,4,7,10-tetraazadecane.

5. A compound having Formula XVI: 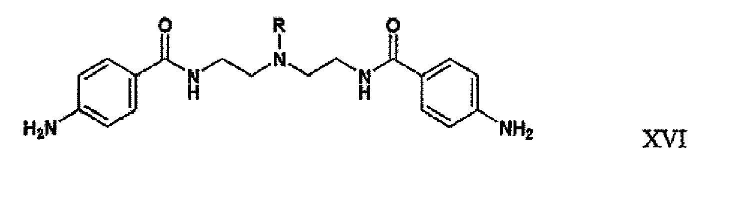 wherein R is H or has Formula XVII:

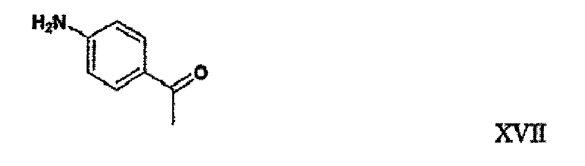

6. A method of making a compound of claim 2, comprising reducing pentaerythritol-(4,3)-ethoxylate-tetrakis(4-nitrobenzoate) to form the compound of claim 2.

7. A method of making a compound of claim 3, comprising reacting pentaerythritol ethoxylate (EO/OH) (3,4) with p-nitrobenzoyl chloride in the presence of pyridine, to form the compound of claim 3.

8. A method of making a compound of claim 2, comprising reacting pentaerythritol ethoxylate (EO/OH) (3,4) with p-aminobenzoic acid in the presence of 2-ethylhexanol and titanium (IV) isopropoxide, and distilling the excess 2-ethylhexanol to form the compound of claim 2.

9. A polyurea cross-linked with one or more of pentaerythritol-(4,3)-ethoxylatetetrakis(4-aminobenzoate), 1,10-di(4-aminophenylcarbonyl)-1,4,7,10-tetraazadecane, 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerythritol tetrakis(3-aminobenzoate), pentaerythritol tetrakis(4-aminobenzoate), or derivatives thereof.

10. A method of making a cross-linked polyurea, comprising combining polyurea reaction products with a cross-linking agent comprising one or more of pentaerythritol-(4,3)-ethoxylate-tetrakis(4-aminobenzoate), 1,10-di(4-aminophenylcarbonyl)-1,4,7,10-tetraazadecane), 1,4,7-tri(4-aminophenylcarbonyl)-1,4,7-triazaheptane, 1,7-di(4-aminophenylcarbonyl)-1,4,7-triazaheptane, pentaerythritol tetrakis(2-aminobenzoate), pentaerythritol tetrakis(3-aminobenzoate), pentaerythritol tetrakis(4-aminobenzoate), and curing to form the cross-linked polyurea.

11. A method of making the compound of claim 4, comprising reacting triethylenetetramine with methyl 4-aminobenzoate in the presence of a lower alcohol (C₁₋₈) solvent to form the compound of claim 4.

12. A method of making the compound of claim 5, comprising reacting dietheylenetriamine with methyl 4-aminobenzoate in the presence of a lower alcohol (C₁₋₈) solvent to form the compound of claim 5.
